# EUROPEAN PATENT APPLICATION

(11) **EP 4 474 814 A1**
(43) Date of publication of application: **11.12.2024**
(21) Application number: 23315239.6
(22) Date of filing: 05.06.2023
(51) Int. Cl.: G01N 33/00, G06N 20/00, G16C 20/20

(54) **DATA AUGMENTATION FOR AN ELECTRONIC NOSE**

(71) Applicant: Aryballe, 38000 Grenoble (FR)
(72) Inventor: Singh, Krishna Kant, 38000 Grenoble (FR)
(74) Representative: Schuffenecker, Thierry

(57) **Abstract**

There is disclosed a computer-implemented method for electronic nose sensorgram data augmentation comprising the steps of: receiving a sensorgram; generating multiple temporal signatures of the sensorgram; generating multiple spatial signatures of the sensorgram; combining the temporal and spatial signatures to obtain an augmented signature database. Developments describe the obtention of a database of augmented signatures and features ; the handling of drifted and drift-removed signatures ; the interpolation of signatures ; the use of a dimensionality reduction mechanism comprising one or more of principal component analysis, t-distributed stochastic neighbor embedding, auto-encoder and/or a variational-autoencoder ; the use of a generative adversarial network, the training of machine learning models by using augmented signature data.

## Description

### Technical field

The described examples are related to signal processing.

More specifically, the described examples describe computer systems based on specific computational models and investigating or analyzing materials by determining their chemical or physical properties and recognition of data; presentation of data; record carriers; or handling record carriers and electric digital data processing and image data processing or generation.

### Background Art

An "odor" (American English) or "odour" (British English) is caused by Volatile Organic Compounds (VOCs) and/or molecules emitted by objects in the environment, which can be found in low concentrations in the air that humans and animals can perceive by their sense of smell. Other terms with different connotations can be used e.g., "smell", "scent", "aroma", "fragrance", "perfumes", etc.

An electronic nose is a sophisticated sensing device that emulates the human olfactory system to detect and analyze odors or volatile compounds in the surrounding environment. It consists of an array of chemical sensors that respond to specific odor molecules or volatile compounds. When exposed to an odor, the sensors generate a unique pattern of responses, known as a sensorgram. The sensorgram represents the sensor responses over time, providing a characteristic fingerprint of the volatile compound. This sensorgram data is then processed and analyzed using various algorithms and techniques to identify and classify the odor, quantify its concentration, and distinguish it from other odors.

Data scarcity is a significant challenge faced in the field of electronic nose technology. Electronic noses are designed to mimic the olfactory capabilities of humans, enabling the detection and identification of various odors and volatile compounds. However, obtaining a large and diverse dataset of sensorgram (and signatures) samples for building a machine learning system (for training and validation purposes) is often a cumbersome task. The limited availability of labeled sensorgram data hampers the development and optimization of robust and accurate ML systems for electronic noses. The scarcity of data poses challenges in accurately modeling and capturing the complex relationships between sensor responses and the corresponding odors or chemical compounds. Furthermore, the lack of diverse data can result in poor generalization and limited applicability of electronic noses across various environments and odor sources. Here we present methods based on sampling, data augmentation and the utilization of generative models to overcome this obstacle and to expand the available data and improve the performance and reliability of electronic nose systems in real-world scenarios.

In nature, there are millions of volatile organic compounds. Generating their odor profile with an electronic nose is very challenging. Moreover, generating odor profiles of mixtures of VOCs is even more challenging.

Today, known methods use molecular modeling and deep learning-based approaches to build predictive models that consider signatures of pure VOCs. These methods work fine for liquid phases, but not for gas phases (for which problems are significantly more difficult to handle)

The article entitled "SMILE to smell" was published on January 15, 2021 in J. Chem. Inf. Model. 2021, 61, 2, 676-688 converts SMILES (simplified molecular-input line-entry system values) into images and uses CNN networks. According to the article, finding the relationship between the structure of an odorant molecule and its associated smell has always been an extremely challenging task. The major limitation in establishing the structure-odor relation is the vague and ambiguous nature of the descriptor-labeling, especially when the sources of odorant molecules are different. With the advent of deep networks, data-driven approaches have been substantiated to achieve more accurate linkages between the chemical structure and its smell. In this study, the deep neural network (DNN) with physicochemical properties and molecular fingerprints (PPMF) and the convolution neural network (CNN) with chemical-structure images (IMG) are developed to predict the smells of chemicals using their SMILES notations. A data set of 5185 chemical compounds with 104 smell percepts was used to develop the multilabel prediction models. The accuracies of smell prediction from DNN + PPMF and CNN + IMG (Xception based) were found to be 97.3 and 98.3%, respectively, when applied on an independent test set of chemicals. The deep learning architecture combining both DNN + PPMF and CNN + IMG prediction models is proposed, which classifies smells and may help understand the generic mechanism underlying the relationship between chemical structure and smell perception.

This approach is interesting but presents limitations. Inter alia, it leads to relatively bad results for odor profiles predictions.

The blog article entitled *"*Digitizing Smell: Using Molecular Maps to Understand Odor" published on September 6th 2022 by Richard C. Gerkin and al., at Google Research teaches how to use graph neural network (GNN) models but nothing more.

The patent document US20220139504 discloses systems and methods for predicting olfactory properties of a molecule. One example method includes obtaining a machine-learned graph neural network trained to predict olfactory properties of molecules based at least in part on chemical structure data associated with the molecules. The method includes obtaining a graph that graphically describes a chemical structure of a selected molecule. The method includes providing the graph as input to the machine-learned graph neural network. The method includes receiving prediction data descriptive of one or more predicted olfactory properties of the selected molecule as an output of the machine-learned graph neural network. The method includes providing the prediction data descriptive of the one or more predicted olfactory properties of the selected molecule as an output. This approach presents limitations.

### Summary

The present document relates to the technical field of data augmentation for electronic nose systems. Electronic noses are devices designed to mimic the olfactory system and detect and identify odors in various applications. However, due to the limited availability and diversity of odor samples, there is a scarcity of data for training and fine-tuning electronic nose systems. This scarcity poses a significant challenge in developing accurate and robust odor detection algorithms. Embodiments address this challenge by providing novel methods and systems for data augmentation in electronic nose systems. These techniques involve generating multiple signatures from a given sensorgram, incorporating temporal and spatial information, compensating for drift effects, handling different backgrounds and concentrations, as well as utilizing generative artificial intelligence models. By augmenting the available data, embodiments enable improved training, validation, and calibration of electronic nose systems.

One of the biggest challenges with electronic noses is the scarcity of available data. Unlike other sensors such as cameras or microphones, electronic noses require direct contact with the substance being detected, which limits the types and amount of data that can be collected. Additionally, odor samples can be expensive, time-consuming, and difficult to obtain, particularly for rare or hazardous substances. This scarcity of data poses a significant challenge for developing accurate and reliable electronic nose systems.

There is disclosed a computer-implemented method for electronic nose data augmentation comprising the steps of: receiving a sensorgram; generating multiple temporal signatures of the sensorgram; generating multiple spatial signatures of the sensorgram; combining the temporal and spatial signatures to obtain an augmented signature database. Developments describe the obtention of a database of augmented signatures and features ; the handling of drifted and drift-removed signatures ; the interpolation of signatures ; the use of a dimensionality reduction mechanism comprising one or more of principal component analysis, t-distributed stochastic neighbor embedding, auto-encoder and/or a variational-autoencoder ; the use of a generative adversarial network, the training of machine learning models by using augmented signature data.

Electronic noses are advantageous for environmental monitoring, food quality control, or even medical diagnosis. By using data augmentation, machine learning models trained on e-nose data can be improved, leading to more accurate and reliable gas and odor detection and identification.

In the context of an electronic nose, the term "signature" refers to a consolidated representation of the sensorgram data that captures the distinctive characteristics of an odor or volatile compound. The sensorgram, which is a temporal sequence of sensor responses, is transformed or processed to derive a more condensed and informative signature. Most common approach to generate a signature from a sensorgram is by taking the average of the sensor responses over time. This method involves calculating the mean value of each sensor's response across the entire or defined duration of the sensorgram.

The average-based signature provides a compact representation of the overall sensor response pattern over time. It captures the central tendency of the sensorgram data and smooths out any short-term fluctuations or noise. This approach is particularly useful when the sensor responses exhibit consistent trends or when the focus is on the general characteristics of the odor rather than specific temporal dynamics.

There is disclosed a method for electronic nose sensorgram data augmentation comprising the steps of: receiving a sensorgram; generating multiple temporal signatures of the sensorgram; generating multiple spatial signatures of the sensorgram; combining the temporal and spatial signatures to obtain an augmented sensorgram.

The method allows for more accurate and reliable electronic nose sensorgram data by combining temporal and spatial signatures, providing a more comprehensive analysis of the sensorgram. By generating multiple temporal signatures of the sensorgram, the method increases the sensitivity and specificity of the electronic nose, allowing for more precise detection and analysis of volatile organic compounds. The extraction of features from the received sensorgram allows for selective detection and analysis of specific compounds, improving the accuracy of the electronic nose in detecting and identifying target compounds.

In a development, the method further comprises extracting one or more features from the received sensorgram, generating multiple temporal and spatial signatures of the one or more extracted features, and combining the temporal and spatial signatures to obtain an augmented sensorgram.

By generating signatures associated with one or more baselines, the method allows for the identification and removal of baseline noise, increasing the accuracy and reliability of the electronic nose in detecting and identifying target compounds. The use of water or humidity as a baseline allows for the detection of water and moisture content in the tested sample, which can be useful in various industries such as food and pharmaceuticals. By combining temporal and spatial signatures for the baselines, the method allows for a more comprehensive analysis of the sample, providing more accurate identification and removal of baseline noise.

In a development, the method further comprises generating signatures associated with one or more baselines, with or without a presence of one or more volatile organic compounds.

The method allows for the identification and removal of background noise caused by signal drift, which can improve the sensitivity and specificity of the electronic nose sensorgram data. By applying Principal Component Analysis to the sensorgram, the method can identify the component that exhibits the highest drift, providing insight into the source of the background noise. The use of a drifted signature can help to identify and remove signal drift, improving the accuracy and reliability of the electronic nose sensorgram data.

In a development, a baseline is water or humidity.

The method allows for more comprehensive analysis of the sensorgram data by generating multiple temporal and spatial signatures of the extracted features. By combining temporal and spatial signatures, the method provides more accurate and reliable detection and identification of volatile organic compounds in the tested sample. The use of the extracted features allows for selective detection and analysis of specific compounds, increasing the specificity and sensitivity of the electronic nose sensorgram data.

In a development, the method further comprises: generating a drifted signature of a sensorgram, by: applying a Principal Component Analysis to the sensorgram, selecting the component that exhibits the highest drift, sampling the sensorgram in a direction of the selected component to obtain a drifted signature.

The method allows for more comprehensive analysis of the sensorgram data by combining temporal and spatial signatures to obtain an augmented sensorgram. The use of the augmented sensorgram improves the accuracy and reliability of the electronic nose in detecting and identifying volatile organic compounds in the tested sample. By combining temporal and spatial signatures, the method provides valuable insights into the chemical composition of the tested sample, allowing for more detailed analysis and identification of the target compounds.

In a development, the method further comprises: applying a Principal Component Analysis to the sensorgram, selecting a component that exhibits the highest drift, and subtracting the drift component from the sensorgram to obtain a drift-removed signature.

The use of Principal Component Analysis allows for efficient identification of the sensorgram component with the highest drift, leading to accurate removal of drift from the sensorgram. Removal of drift from the sensorgram allows for more accurate analysis and identification of the target substance, leading to improved sensor performance. The drift-removed signature obtained through this method can be used as a template for future sensorgrams, streamlining the analysis process and improving sensor longevity.

In a development, the method further comprises: generating a drifted signature, generating a drift-removed signature, combining the received, drifted, and/or drift-removed signatures to obtain an augmented sensorgram.

The combination of original, drifted, and drift-removed signatures creates an augmented sensorgram that provides a more comprehensive and accurate analysis of the target substance. The use of multiple signatures allows for comparison and cross-validation of results, increasing the reliability and accuracy of the sensor. The augmented sensorgram can be adjusted and customized for specific applications, improving the versatility and usefulness of the sensor.

In a development, the method further comprises: receiving a sensorgram, applying a non-linear transformation to the received sensorgram, generating multiple temporal and spatial signatures of the transformed sensorgram, and combining the temporal and spatial signatures to obtain an augmented sensorgram.

The use of a non-linear transformation provides increased accuracy and sensitivity in the analysis of the sensorgram. The generation of multiple spatial and temporal signatures allows for more precise and detailed analysis of the target substance. The combination of these signatures creates an augmented sensorgram with enhanced accuracy and sensitivity, improving overall sensor performance.

In a development, the method further comprises: interpolating signatures at different concentrations, by: generating signatures of an odor at multiple concentrations, selecting a reference concentration, interpolating the signatures at the reference concentration using a regression algorithm to obtain an interpolated signature.

Interpolating signatures at different concentrations allows for more accurate analysis of targets across a broader range of concentrations than previously possible. The use of a regression algorithm facilitates efficient and accurate interpolation, reducing the time and resources required for analysis. The interpolated signature provides a more accurate representation of the target substance at a variety of concentrations, improving sensor performance and reliability.

In a development, the method further comprises: mapping the sensorgram data to a lower dimension using a dimensionality reduction mechanism, sampling the lower-dimensional data to obtain multiple temporal and spatial signatures, combining the temporal and spatial signatures to obtain an augmented sensorgram.

The dimensionality reduction mechanism reduces noise and redundancy in the sensorgram data, improving signal-to-noise ratio and increasing accuracy. Sampling the lower-dimensional data provides a more efficient and targeted analysis of the sensorgram, reducing the time and resources required for analysis. Combining the temporal and spatial signatures of the lower-dimensional data creates an augmented sensorgram with enhanced accuracy and sensitivity, improving overall sensor performance.

In a development, the dimensionality reduction mechanism comprises one or more of the mechanisms comprising: principal component analysis, t-distributed stochastic neighbor embedding, auto-encoder and/or a variational-autoencoder.

By utilizing the principal component analysis mechanism, the dimensionality reduction mechanism is able to effectively identify and retain relevant information from high-dimensional data sets, resulting in improved data processing efficiency and accuracy. The t-distributed stochastic neighbor embedding mechanism is able to preserve the internal structure of the data, allowing for more accurate clustering and visualization of high-dimensional data sets. The combination of multiple mechanisms, such as auto-encoder and variational autoencoder, allows for a more comprehensive approach to dimensionality reduction, resulting in improved data representation and analysis.

In a development, the method further comprises: generating additional sensorgram data using the trained generative artificial intelligence model, generating multiple temporal and/or spatial signatures from the original and generated sensorgram data, and combining the temporal and/or spatial signatures to obtain an augmented sensorgram.

By generating additional sensorgram data using a trained generative artificial intelligence model, the accuracy and completeness of the data set is improved, allowing for more accurate pattern recognition and analysis. The generation of multiple temporal and/or spatial signatures from both the original and generated data sets provides a more complete and accurate representation of the underlying data patterns, resulting in improved pattern recognition and analysis. The combination of temporal and spatial signatures to obtain an augmented sensorgram allows for more accurate and comprehensive representation of the underlying data patterns, resulting in improved pattern recognition and analysis.

In a development, the a generative adversarial network comprises one or more of: a conditional generative adversarial network, a deep convolutional generative adversarial network, a self-attention generative adversarial network, a variational autoencoder generative adversarial network, a transformer generative adversarial network, a flow-generative adversarial network, a Hinge loss generative adversarial network, a least squares generative adversarial network, a Wasserstein generative adversarial network, a bidirectional generative adversarial network and/or a cycle-generative adversarial network.

The use of a variety of generative adversarial network mechanisms, such as conditional generative adversarial network, self-attention generative adversarial network, and flow-generative adversarial network, allows for a more versatile and comprehensive approach to generating artificial data sets, resulting in improved accuracy and completeness of the generated data. The use of generative adversarial network mechanisms, such as Wasserstein generative adversarial network and least squares generative adversarial network, allows for improved stability and convergence of the artificial data generation process. The combination of multiple generative adversarial network mechanisms, such as transformer generative adversarial network and cycle-generative adversarial networks, allows for a more comprehensive and accurate approach to generating artificial data sets, resulting in improved data analysis and pattern recognition.

In a development, the method further comprises: receiving sensorgram data from a sensor. generating additional sensorgram data using the trained generative artificial intelligence model, extracting one or more features for generating multiple temporal and/or spatial signatures from the received sensorgram data and generated sensorgram data, and combining one or more signatures by combining the temporal and/or spatial signatures to obtain an augmented sensorgram.

By extracting features from both the received sensorgram data and generated sensorgram data, a more comprehensive and accurate representation of the underlying data patterns is obtained, resulting in improved data analysis and pattern recognition. The use of generated sensorgram data allows for a more complete and accurate representation of the underlying data patterns, resulting in improved accuracy and completeness of the generated multiple temporal and/or spatial signatures. The combination of temporal and spatial signatures to obtain an augmented sensorgram allows for a more accurate and comprehensive representation of the underlying data patterns, resulting in improved pattern recognition and analysis.

### Brief description of drawings

The present disclosure is illustrated by way of example and not limited to the accompanying figures in which reference numerals indicate similar elements and in which:
FIG. 1 illustrates an embodiment of an electronic nose;
FIG. 2 illustrates different phases in odor detection;
FIG. 3 illustrates different signals associated with different signatures;
FIG. 4 illustrates different signals and associated hardware sensors;
FIG. 5 illustrates the augmentation of a signature;
FIG. 6 illustrates an embodiment;
FIG. 7 illustrates an overview of relationships between VOCs, signatures, odor space, chemical space and smell.

### Detailed description

### Definitions

### Electronic nose

An electronic nose (e-nose) is a device that is designed to mimic the human sense of smell. It can use an array of chemical sensors to detect and identify different odors or volatile organic compounds (VOCs) in the air.

The electronic nose works by exposing the chemical sensors to a sample of air or gas, which contains the odor or VOCs of interest. The sensors then produce a signal that is analyzed to identify the specific odors or VOCs present in the sample. The output of the electronic nose is typically a pattern or signature of the different odors or VOCs present in the sample.

Electronic noses have a wide range of applications in various fields such as food and beverage industry, environmental monitoring, medical diagnosis, and quality control in manufacturing processes. For example, electronic noses can be used to detect the presence of spoilage or contaminants in food products, to monitor air quality in industrial or urban environments, or to diagnose medical conditions based on breath samples.

### Data augmentation

One of the biggest challenges with electronic noses is the scarcity of available data. Unlike other sensors such as cameras or microphones, electronic noses require direct contact with the substance being detected, which limits the types and amount of data that can be collected. Additionally, odor samples can be expensive, time-consuming, and difficult to obtain, particularly for rare or hazardous substances. This scarcity of data poses a significant challenge for developing accurate and reliable electronic nose systems.

Data augmentation in data analysis are techniques used to increase the amount of data by adding slightly modified copies of already existing data or newly created synthetic data from existing data. It acts as a regularizer and helps reduce overfitting when training a machine learning model. It can be related to oversampling in data analysis.

Data augmentation for electronic nose (e-nose) refers to techniques used to increase the size and diversity of a dataset of sensor responses obtained from an electronic nose. An electronic nose is a device that uses an array of gas sensors to detect and identify various gasses and odors. These sensors generate signals that are processed and analyzed to identify the type of gas or odor present.

Data augmentation techniques for e-nose can include adding noise to the sensor signals, artificially creating missing sensor data, or generating new sensor signals using machine learning models. Data augmentation techniques can include simple operations such as flipping, cropping, or rotating images or adding noise to audio files. More advanced techniques can involve generating new data samples using techniques like generative adversarial networks (GANs) or variational autoencoders (VAEs).

The objective of data augmentation in e-nose is to improve the robustness and generalization of machine learning models trained on e-nose data by introducing variations in the training data that reflect real-world conditions.

In a development, data augmentation techniques can comprise one or more of:
- adding noise or perturbations to the input data,
- applying transformations such as rotations or scaling to the input data;
- or generating synthetic data using generative models;
- adding noise to the sensor signals;
- artificially creating missing sensor data;
- generating new sensor signals using machine learning models, using techniques like generative adversarial networks (GANs) or variational autoencoders (VAEs);
- with respect to drawings, flipping, cropping, or rotating images or adding noise to audio files. More advanced techniques can involve generating new data samples;

### Data augmentation by graph augmentation

In a development, data augmentation techniques can comprise a graph augmentation, comprising one or more of:
- adding synthetic nodes: using techniques like random graph generation, and add them to the existing graph to increase its size and complexity;
- adding synthetic edges: synthetic edges can be generated using techniques like link prediction, and add them to the existing graph to connect nodes that were previously disconnected;
- adding node attributes: natural language processing techniques can be used to generate text descriptions of nodes and their properties, and add them as attributes to the corresponding nodes in the graph;
- adding edge attributes: image processing techniques can be used to generate visual features of the edges, and add them as attributes to the corresponding edges in the graph;
- adding subgraphs: graph clustering techniques can be used to identify subgraphs within the original graph, and add them as additional nodes with their own attributes and connections to the rest of the graph.

### Generative adversarial network

A generative adversarial network (GAN) is a class of machine learning frameworks in which two neural networks contest with each other in the form of a zero-sum game, where one agent's gain is another agent's loss. The generative network generates candidates while the discriminative network evaluates them. The contest operates in terms of data distributions. Typically, the generative network learns to map from a latent space to a data distribution of interest, while the discriminative network distinguishes candidates produced by the generator from the true data distribution. The generative network's training objective is to increase the error rate of the discriminative network (i.e., "fool" the discriminator network by producing novel candidates that the discriminator thinks are not synthesized (are part of the true data distribution). A known dataset serves as the initial training data for the discriminator. Training involves presenting it with samples from the training dataset until it achieves acceptable accuracy. The generator is trained based on whether it succeeds in fooling the discriminator. Typically, the generator is seeded with randomized input that is sampled from a predefined latent space (e.g., a multivariate normal distribution). Thereafter, candidates synthesized by the generator are evaluated by the discriminator. Independent backpropagation procedures are applied to both networks so that the generator produces better samples, while the discriminator becomes more skilled at flagging synthetic samples, where one agent's gain is another agent's loss.

### Dimensionality reduction

Dimensionality reduction can comprise one or more of the mechanisms comprising: principal component analysis, t-distributed stochastic neighbor embedding, auto-encoder and/or a variational-autoencoder.

A t-distributed stochastic neighbor embedding designates a system wherein two neural networks contest with each other in the form of a zero-sum game, where one agent's gain is another agent's loss.

An autoencoder is a type of artificial neural network used to learn efficient coding of unlabeled data (unsupervised learning).An autoencoder learns two functions: an encoding function that transforms the input data, and a decoding function that recreates the input data from the encoded representation. The autoencoder learns an efficient representation (encoding) for a set of data

Variational autoencoders are probabilistic generative models that require neural networks as only a part of their overall structure. The neural network components are typically referred to as the encoder and decoder for the first and second component respectively. The first neural network maps the input variable to a latent space that corresponds to the parameters of a variational distribution. In this way, the encoder can produce multiple different samples that all come from the same distribution. The decoder has the opposite function, which is to map from the latent space to the input space, in order to produce or generate data points. Both networks are typically trained together with the usage of the reparameterization trick, although the variance of the noise model can be learned separately.

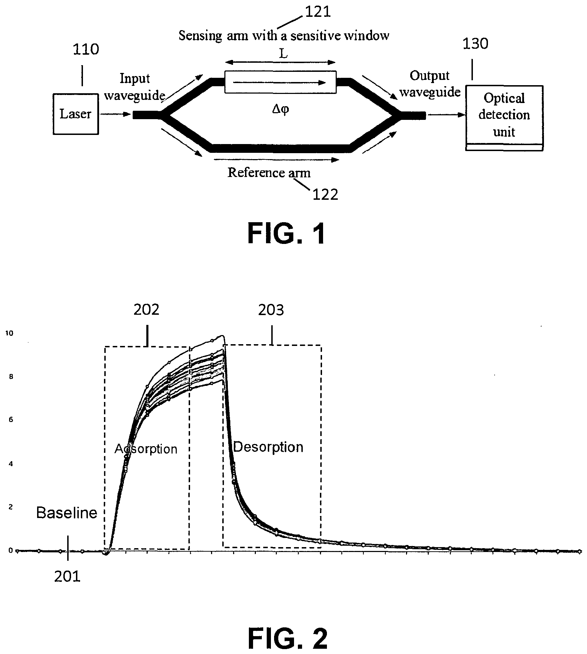

FIG. 1 illustrates an embodiment of an electronic nose.

The structure of an electronic nose can be diverse.

In an embodiment, the "electronic nose" or "analyzing system" comprises a measurement chamber 121 intended to receive the target compounds contained in either gas, liquid or fluid sample, wherein a plurality of separate sensitive sites, each comprising receivers able to interact with the target compounds, is located in said chamber.

In an electronic nose, VOCs (or fluid sample) are generally injected (passively and/or actively) into a chamber 121 with or without inert gas (e.g., argon). Distinct sensor units (of distinct chemical affinity) forming a "multivariate sensor" in the chamber then react to compounds or VOCs or molecules of the fluid sample and from an analog output, a digital output is determined, and further interpreted.

A "sensorgram" is obtained, comprising substantially parallel curves, each curve being the response of one sensor unit to compounds or VOCs. Amplifiers can be used. From said sensorgram, a signature is determined (such as a radar chart), said signature being associated with one or more predefined "odors" (interpretation of the presence of compounds can be post-processed).

Databases can be built, in particular in the form of signatures and/or low-dimensional embeddings, i.e., using metric learning, PCA or trained neural networks.

Principal Component Analysis (PCA) is a technique for analyzing large datasets containing a high number of dimensions/features per observation, increasing the interpretability of data while preserving the maximum amount of information, and enabling the visualization of multidimensional data. PCA is a statistical technique for reducing the dimensionality of a dataset.

In an embodiment, the sensor comprises an optical integrated circuit comprising: a lower layer called substrate; a first coupling means adapted to couple a radiation incident light (laser) 100 to the integrated optical circuit; a directional splitter connected to the first coupling means and configured to separate the light radiation coupled by the first means from the second means coupling to at least one pair of waveguides comprising: a first waveguide known as a sensitive arm 121 in which is propagated a first portion of the light radiation, said sensitive arm 121 being exposed to a first ambient medium and at least one compound to be detected that induces a modification of the local refractive index perceived by the evanescent part of the electromagnetic field of the first portion of the light radiation, and a second waveguide called a reference arm 122 in which is propagated a second portion of the light radiation, an encapsulation layer encapsulating the reference arm, said layer being impermeable to the compound(s) to be detected, so as to be able to detect that the reference arm is exposed only to a second medium the same nature as the first ambient environment, and devoid of said compound to be detected ; a directional combiner combining the first portion of the radiation from said reference arm, called the first transmitted portion, and the second portion of the light radiation from said sensitive arm, called the "sensitive arm" second transmitted portion, to form a transmitted radiation ; second coupling means adapted to couple said radiation; and transmitted to a medium external to the integrated optical circuit; an upper layer called superstrate covering at least the first and the second layers of the optical integrated circuit; second coupling means, the directional separator and the combiner directional and does not cover the sensitive and reference arm, said encapsulation layer being deposited on top of the superstrate.

In an embodiment, the sensor comprises: a laser source 100 configured to emit incident light radiation; an integrated optical circuit; an optical detection system 130 adapted to detect a light radiation; - an integrated optical circuit from the second coupling means and generate a signal representative of the evolution over time of the detected light intensity; a unit for processing the said signal, adapted to determine, from the intensity detected, the evolution over time of the phase shift between the first portion and the second portion transmitted and the second portion transmitted.

Sensors in modern technology are becoming more and more complex. In some embodiments, an electronic nose can comprise one or more of SPR, MZI, MEMS, CMUT, and/or Carbon Nanotubes.

### Surface Plasmon Resonance (SPR)

Surface plasmon resonance (SPR) occurs where electrons in a thin metal sheet become excited by light that is directed to the sheet with a particular angle of incidence, and then travel parallel to the sheet. Assuming a constant light source wavelength and that the metal sheet is thin, the angle of incidence that triggers SPR is related to the refractive index of the material and even a small change in the refractive index will cause SPR to not be observed. This makes SPR a possible technique for detecting particular substances (analytes) and SPR biosensors have been developed to detect various important biomarkers

### Mach-Zehnder interferometer (MZI)

Response signals of a MZI transducer (Mach-Zehnder Interferometer) are signals modulated (by an optical stage) in frequency proportional to the difference in step between the wave in the reference guide and the wave in the measuring guide. This difference allows the reconstruction of an odor sensorgram. In variants, the multivariate sensor comprises a Fizeau interferometer or a Fabry-Pérot interferometer or a Jamin interferometer or a Ramsey-Bordé interferometer.

### MEMS

MEMS (Micro-electro-mechanical systems) incorporates both electronic and moving parts. MEMS are made up of components between 1 and 100 micrometers in size, and MEMS devices generally range in size from 20 micrometers to a millimeter, although components arranged in arrays (e.g., digital micromirror devices) can be more than 1000 mm2. They usually consist of a central unit that processes data (an integrated circuit chip such as microprocessor) and several components that interact with the surroundings (such as microsensors)

### Capacitive Micromachined Ultrasonic Transducer, acronym CMUT

In an embodiment, the multivariate sensor comprises a Capacitive Micromachined Ultrasonic Transducer, acronym CMUT. CMUTs are the transducers where the energy transduction is due to change in capacitance. CMUTs are constructed on silicon using micromachining techniques. A cavity is formed in a silicon substrate, and a thin layer suspended on the top of the cavity serves as a membrane on which a metallized layer acts as an electrode, together with the silicon substrate which serves as a bottom electrode. In an embodiment, the multivariate sensor can comprise a large number of CMUTs. In particular, CMUT-on-CMOS technology along with a flip-chip process can allow integration of CMUTs with front-end electronics.

### Carbon Nanotubes

Aligned Carbon Nanotubes (CNTs) can be grown at low temperature (250 °C) using Plasma Enhanced Chemical Vapor Deposition (PECVD). CNTs can then be used to identify VOCs in an improved sensitivity condition to gas at room temperature. Sensitivity and stability of carbon nanotube sensors can be improved via growing Titanium Dioxide Nanowires (TiO2-NW) on the surface of CNTs through a hydrothermal method. A threefold increase in sensitivity and a 30-second decrease in response time can be observed as a result of the CNT-TiO2 sensor compared to a pristine CNT sensor.

### Other sensor embodiments

In an advantageous embodiment, peptides are used. Peptides are short chains of between two and fifty amino acids, linked by peptide bonds. Chains of fewer than ten or fifteen amino acids are called oligopeptides, and include dipeptides, tripeptides, and tetrapeptides. A "polypeptide" is a longer, continuous, unbranched peptide chain of up to approximately fifty amino acids. Hence, peptides fall under the broad chemical classes of "biological polymers and oligomers", alongside nucleic acids, oligosaccharides, polysaccharides, and others.

In some embodiments, polymers (on silicon or derivative materials like SiO2 or other traditional materials in micromachining MEMS or silicon photonics) can be used. Silicon is a chemical element with the symbol Si and atomic number 14. It is a semiconductor.

In some embodiments, piezo resistance mechanisms can be used. In some embodiments, living cells can be used. In some embodiments, combinations of the above means can be used, e.g., a combination of peptides and polymers. Depending on use cases (i.e., general purpose or specific detection), the number and/or types of sensor units are diverse.

FIG. 2 illustrates different phases in odor detection.

An "electronic nose" (irrespective of sensor technology) produces a signal 200 when its sensors interact with an odor.

In the absence of gas, the baseline 201 is measured. Absorption 202 is generally the first phase, followed by equilibrium and then desorption 203. In rare cases, the cycle can start by a desorption phase 203 (for example by eating). Adsorption 202 generally occurs exponentially, then a plateau is obtained, then a desorption 203 occurs.

Most often, after the equilibrium phase, the obtained signal is transformed into a *signature* by performing the integration over time.

In other words, a sensor unit response generally comprises three phases. At first, only air or neutral inert gas is present: sensor units exhibit a "baseline" level 201. Then, VOCs start coupling with reagents and the absorption phase 202 starts. At a certain moment, after a specific time duration, an "equilibrium" is obtained. Then, triggered by a purge (active removal of VOCs, e.g., caused by injecting air and/or other ways such as heating), the phase of desorption 203 starts. Molecules leave sensor units (not necessarily symmetrical to the absorption phase). In some situations, the desorption phase can be the first and sometimes only step.

Different baselines designate different backgrounds. Signatures of mixtures can be obtained by linear combination.

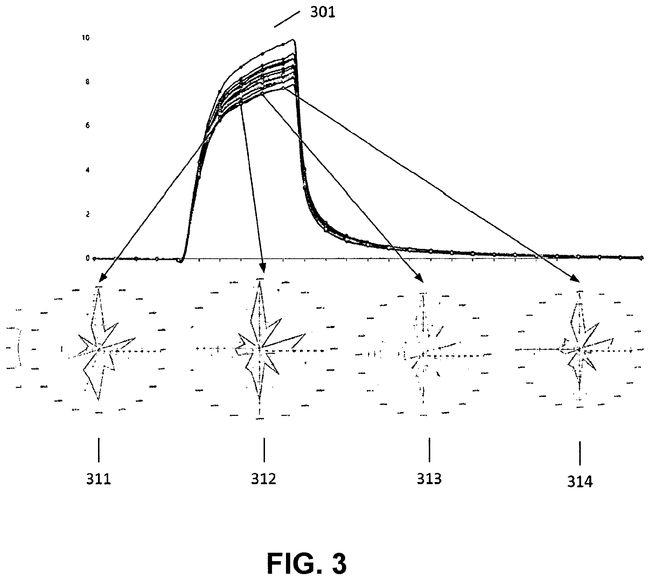

FIG. 3 illustrates different signals associated with different signatures.

Different signals 301 can lead to diverse signatures 311, 312, 313 and 314.

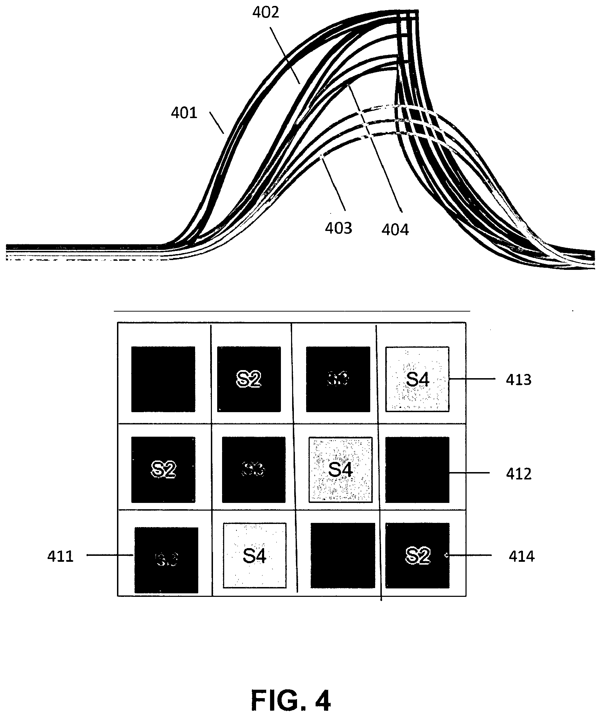

FIG. 4 illustrates different signals and associated hardware sensors;

Different signals 401, 402, 403 and 404 are sensed by sensors S1, S2, S3 and S4 arranged in a matrix.

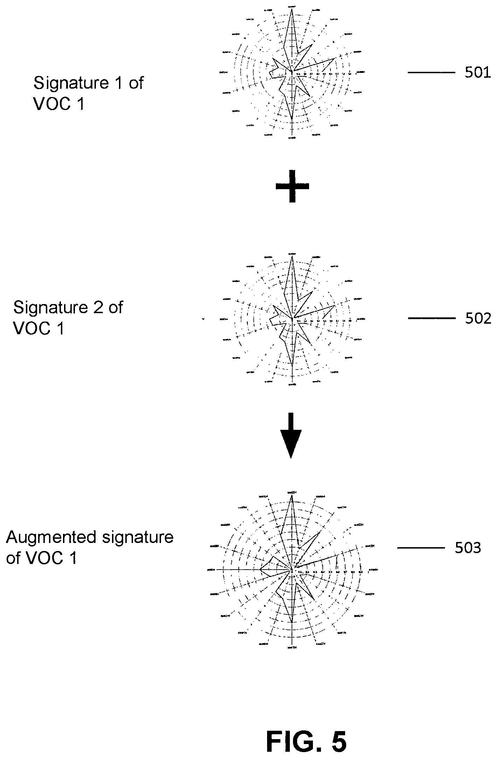

FIG. 5 illustrates the augmentation of a signature;

The first signature 501 of a given VOC is added to the signature 502 of a given VOC thereby obtaining an augmented signature 503 of the given VOC.

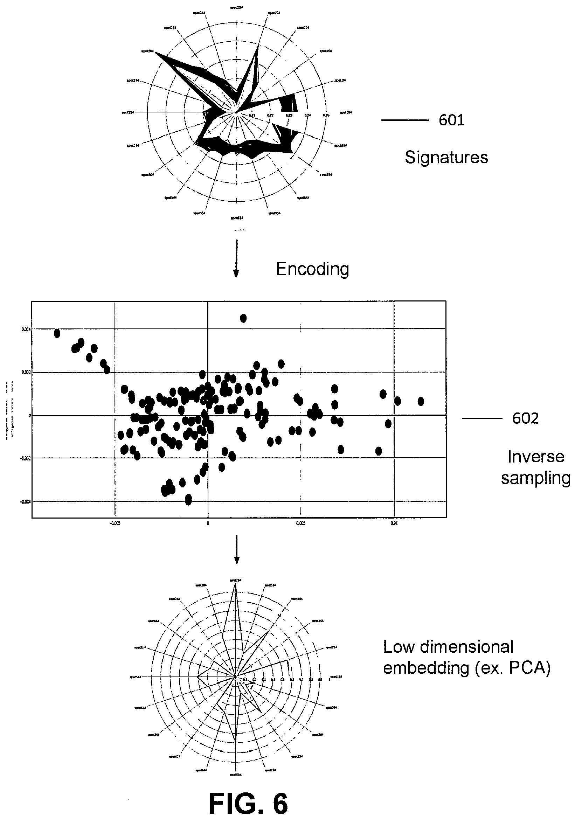

FIG. 6 illustrates an embodiment.

One or more signatures 601 can be encoded by inverse sampling 602 and determine a low-dimensional embedding (for example a PCA)

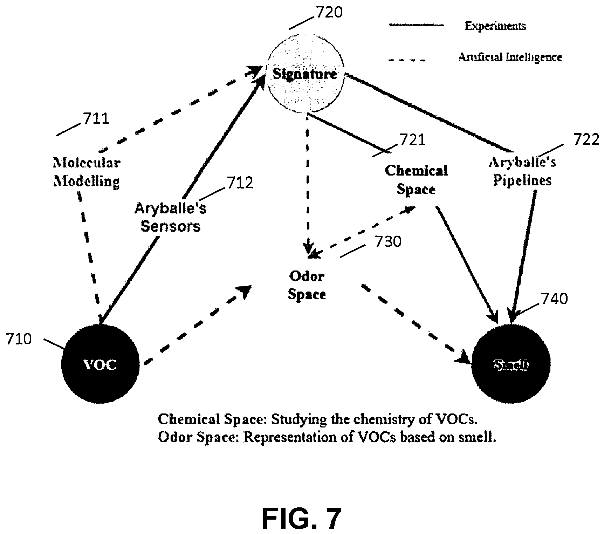

FIG. 7 illustrates an overview of relationships between VOCs, signatures, odor space, chemical space and smell.

VOC 710 designates Volatile Organic Compounds. VOCs can be represented as SMILES in theoretical and computational chemistry. These smiles are then used to identify the structure-to-activity relationship also known as QSAR (Quantitative Structural Activity Relationship). In some embodiments, the activity can be the smell associated with a VOC.

Latent low-dimensional space can show a fuzzy relationship between SMILES and smell is known as Odor-space 730.

Most theoretical work is designed for determining relationships between VOC (710), Odor Space 730 and smell 740

In reality, most often, emitted VOCs 710 are not known (so as the composition of VOCs in the gas phase). For example, VOCs are released by wine or perfume. In general, we do not know these VOCs.

Instruments such as electronic nose convert emitting VOC (or mixture of VOCs) into a numerical signal. We refer to this numerical signal as a Signature 720.

Sensors 712 are biological-based multivariate sensors that convert the binding of VOCs with peptide to numerical data using optical methods. For a VOC, the ranking of sensors is directly associated with binding strength.

Molecular modeling 711 approaches such as docking and molecular dynamics can be used to estimate the binding energy of a VOC with a given peptide sensor. Drawbacks comprise the fact that all the biological molecular modeling simulation is validated in the liquid phase and the electronic nose works in the gas phase.

Signatures to smell 722 according to some embodiments can determine a mapping to convert signatures to smell.

The path 721 (Signature-Chemical Space-Odor) can require a plurality of processes: a) mapping signature to Chemical functional group (example: alcohol, aldehyde, ketone etc.), and mapping Chemical space to odor. It is also possible to map Chemical-space to Odor-space.

This specification uses the term "configured" in connection with systems and computer program components. For a system of one or more computers to be configured to perform particular operations or actions means that the system has installed on it software, firmware, hardware, or a combination of them that in operation cause the system to perform the operations or actions. For one or more computer programs to be configured to perform particular operations or actions means that the one or more programs include instructions that, when executed by data processing apparatus, cause the apparatus to perform the operations or actions.

A computer program, which may also be referred to or described as a program, software, a software application, an app, a module, a software module, a script, or code, can be written in any form of programming language, including compiled or interpreted languages, or declarative or procedural languages; and it can be deployed in any form, including as a stand-alone program or as a module, component, subroutine, or other unit suitable for use in a computing environment. A program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub-programs, or portions of code. A computer program can be deployed to be executed on one computer or on multiple computers that are located at one site or distributed across multiple sites and interconnected by a data communication network.

Computers suitable for the execution of a computer program can be based on general or special purpose microprocessors or both, or any other kind of central processing unit. Generally, a central processing unit will receive instructions and data from a read-only memory or a random-access memory or both. The essential elements of a computer are a central processing unit for performing or executing instructions and one or more memory devices for storing instructions and data. The central processing unit and the memory can be supplemented by, or incorporated in, special purpose logic circuitry. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device, e.g., a mobile telephone, a personal digital assistant (PDA), a mobile audio or video player, or a portable storage device, e.g., a universal serial bus (USB) flash drive, to name just a few.

Processing can be performed locally and/or remotely (distance resources). Connectivity can be continuous or intermittent. In some embodiments, network access can be available and processing (inference) can be performed remotely (e.g., elastic cloud along local processor in the smartphone). In some embodiments, it may well be that no connection is accessible (e.g., loT device): databases can be used locally (low embeddings). Local databases or neural networks also enable fast response time. Data processing apparatus for implementing machine learning models can also include, for example, special-purpose hardware accelerator units for processing common and compute-intensive parts of machine learning training or production, i.e., inference, workloads.

Various display devices can be used. The display can be placed on a smartwatch, or in a smartphone. Display means also can comprise an opaque virtual reality headset or a semi-transparent augmented reality headset or a headset with configurable transparency, projectors (pico-projectors for example, or video-projectors to project simulation scenes) or a combination of such devices. The display can be placed in or on a "head-mounted display", a "wearable computer", "glasses", a headset, etc. The information displayed can be entirely virtual (displayed in the individual helmet), entirely real (e.g., projected on the flat surfaces available in the real cockpit environment) or a combination of both (partly a virtual display superimposed or merged with reality and partly a real display via projectors).

The system implementing steps of the method can comprise one or more displays in a man-machine interface. Human-machine interaction is complex. A feedback loop may be "closed" i.e., inaccessible to human control (it is executed by the machine). It can be "open" (e.g., display step in a human-machine interface, validation or any other human confirmation system). Different embodiments can lead to different implementations by closing, respectively opening, one or more feedback loops. In this way, the process (which may be "artificial intelligence") can be interpreted as "transparent" in the sense of controllable. The display can relate to intermediate computation results, information about root causes, and/or the computation context. In this way, embodiments can be considered "explainable artificial intelligence".

## Claims

**1.** A computer-implemented method for electronic nose sensorgram data augmentation comprising the steps of:
- receiving a sensorgram;
- generating multiple temporal signatures of the sensorgram;
- generating multiple spatial signatures of the sensorgram; and
- combining the temporal and spatial signatures to obtain an augmented signature database.

**2.** The computer-implemented method of Claim 1, further comprising:
- extracting one or more features from said received sensorgram;
- generating multiple temporal and spatial signatures of said one or more extracted features;
- combining the temporal and spatial signatures and features to obtain an augmented signatures and features database.

**3.** The computer-implemented method of any preceding Claim, further comprising generating signatures associated with one or more baselines, with or without a presence of one or more volatile organic compounds.

**4.** The computer-implemented method of Claim 3, wherein a baseline is water or humidity.

**5.** The computer-implemented of any preceding Claim, further comprising:
- generating a drifted signature of a sensorgram, by:
- applying a Principal Component Analysis to the sensorgram;
- selecting the component that exhibits the highest drift;
- sampling the sensorgram in the direction of the selected component to obtain a drifted signature.

**6.** The computer-implemented method of any preceding Claim, further comprising:
- applying a Principal Component Analysis to the sensorgram;
- selecting a component that exhibits the highest drift;
- subtracting the drift component from the sensorgram to obtain a drift-removed signature.

**7.** The computer-implemented method of Claim 5 and 6, comprising:
- receiving a sensorgram;
- generating a drifted signature;
- generating a drift-removed signatures;
- combining the received, drifted, and/or drift-removed signatures to obtain an augmented signatures database.

**8.** The computer-implemented method of any preceding Claim, further comprising:
- receiving a sensorgram;
- applying a non-linear transformation to the received sensorgram;
- generating multiple temporal and spatial signatures of the transformed sensorgram;
- combining the temporal and spatial signatures to obtain an augmented signature database.

**9.** The computer-implemented method of any preceding Claim, further comprising:
- interpolating signatures at different concentrations, by:
- generating signatures of an odor at multiple concentrations;
- selecting a reference concentration;
- interpolating the signatures at the reference concentration using a regression algorithm to obtain an interpolated signature.

**10.** The computer-implemented method of any preceding Claim, comprising:
- receiving a sensorgram;
- mapping the sensorgram data to a lower dimension using a dimensionality reduction mechanism;
- sampling the lower-dimensional data to obtain multiple temporal and spatial signatures;
- combining the temporal and spatial signatures to obtain a signature database.

**11.** The computer-implemented method of Claim 10, wherein the dimensionality reduction mechanism comprises one or more of the mechanisms comprising: principal component analysis, t-distributed stochastic neighbor embedding, auto-encoder and/or a variational-autoencoder.

**12.** The computer-implemented method of any preceding Claim, using a generative adversarial network, comprising the steps of:
- training a generative artificial intelligence model on a set of sensorgram data;
- generating additional sensorgram data using the trained generative artificial intelligence model;
- generating multiple temporal and/or spatial signatures from the original and generated sensorgram data;
- combining the temporal and/or spatial signatures to obtain signature database

**13.** The computer-implemented method of Claim 12, wherein the a generative adversarial network comprises one or more of: a conditional generative adversarial network, a deep convolutional generative adversarial network, a self-attention generative adversarial network, a variational autoencoder generative adversarial network, a transformer generative adversarial network, a flow-generative adversarial network, a Hinge loss generative adversarial network, a least squares generative adversarial network, a Wasserstein generative adversarial network, a bidirectional generative adversarial network and/or a cycle- generative adversarial network.

**14.** The computer-implemented method of any preceding Claim, comprising:
- training a generative artificial intelligence model on a set of sensorgram data;
- receiving sensorgram data from a sensor;
- generating additional sensorgram data using the trained generative artificial intelligence model;
- extracting one or more features for generating multiple temporal and/or spatial signatures from the received sensorgram data and generated sensorgram data;
- combining one or more signatures by combining the temporal and/or spatial signatures to obtain an augmented sensorgram.

**17.** The computer-implemented method of any preceding Claim, comprising:
- generating a new signature by linear combination of signatures or/and generating a new sensorgram by linear combination of sensorgrams; and
- extracting temporal and/or spatial signatures.

**18.** A system configured to perform the steps of the method according to any claims 1 to 17.

**19.** The system of Claim 18, wherein the electronic nose is augmented with one or more sensors, selected from the group comprising : humidity sensor, temperature sensor, photoionized sensor, or gas-specific sensor.

**20.** The system of Claim 19, wherein machine learning models are trained using augmented signature data.
